(19) Europäisches Patentamt
European Patent Office
Office européen des brevets

(11) EP 1 307 202 B1

(12) EUROPEAN PATENT SPECIFICATION

(45) Date of publication and mention of the grant of the patent:
**17.05.2006 Bulletin 2006/20**

(21) Application number: **01941430.9**

(22) Date of filing: **20.06.2001**

(51) Int Cl.:
***A61K 31/513*** *(2006.01)* ***A61P 13/10*** *(2006.01)*

(86) International application number:
**PCT/SE2001/001420**

(87) International publication number:
**WO 2001/097811 (27.12.2001 Gazette 2001/52)**

(54) **METHOD FOR THE TREATMENT OF OVERACTIVE BLADDER**

METHODE ZUR BEHANDLUNG DER HYPERAKTIVEN BLASE

METHODE DE TRAITEMENT D'UNE SURACTIVITE DE LA VESSIE

(84) Designated Contracting States:
**AT BE CH CY DE DK ES FI FR GB GR IE IT LI LU MC NL PT SE TR**

(30) Priority: **22.06.2000 GB 0015246**

(43) Date of publication of application:
**07.05.2003 Bulletin 2003/19**

(73) Proprietor: **AstraZeneca AB**
**151 85 Södertälje (SE)**

(72) Inventors:
- **BIALECKI, Russell A.**
  **Wilmington, DE 19850-5437 (US)**
- **RUMSEY, William**
  **Wilmington, DE 19850-5437 (US)**
- **RUSSELL, Keith**
  **Wilmington, DE 19850-5437 (US)**
- **BERNSTEIN, Peter,**
  **AstraZeneca Wilmington**
  **Wilmington, DE 19850-5437 (US)**
- **AHARONY, David**
  **Wilmington, DE 19850-5437 (US)**

(56) References cited:
**WO-A1-00/02859** **WO-A1-00/20003**
**WO-A1-00/34243** **WO-A1-95/12577**
**WO-A1-96/28158** **WO-A2-00/25766**
**US-A- 6 008 223**

- **CARLO ALBERTO MAGGI ET AL.: 'In vivo and in vitro pharmacology of SR 48,968, a non-peptide tachykinin NK2 receptor antagonist' EUROPEAN JOURNAL OF PHARMACOLOGY vol. 234, 1993, pages 83 - 90, XP002948169**
- **CARLO ALBERTO MAGGI ET AL.: 'Men 10,627, a novel polycyclic peptide antagonist of tachykinin NK2 receptors' THE JOURNAL OF PHARMACOLOGY AND EXPERIMENTAL THERAPEUTICS vol. 271, no. 3, 1994, pages 1489 - 1500, XP002948170**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

### Field of the Invention:

This invention relates to a method for the treatment and/or prevention of overactive bladder or urinary incontinence and compounds and compositions for the use in the method.

### Background:

Overactive bladder ("OAB") is a term for a syndrome that encompasses urge incontinence, urgency and frequency. Urinary incontinence ("UI") is the involuntary loss of urine that results from an inability of the bladder to retain urine as a consequence of either urge (urge incontinence), or physical or mental stress (stress incontinence).

The normal bladder fills at a physiological rate dictated by the function of the kidneys. The bladder can accommodate large volumes of urine due to the physical properties of the bladder as well as a neural inhibitory system. The inhibitory mechanism is believed to involve inhibition of parasympathetic activity or an increase in sympathetic tone to produce detrusor relaxation and allow filling to occur. During filling the outlet neck of the bladder and urethra are contracted preventing leakage. Voiding or micturition is characterized by a relaxation of the outlet neck and the urethra followed by contraction of the detrusor muscle. When the bladder is empty the detrusor muscle relaxes and the outlet neck and urethra contract to seal off the bladder and maintain continence.

Between 4 and 8% of the total population are estimated to suffer from UI at any point in time, although in most countries, only about 15% of such sufferers are diagnosed. Of those diagnosed only about 70% receive medical treatment. Urge incontinence is more prevalent in the elderly and 80% of the cases are female. Pads and other physical devices are regularly used by a large proportion of incontinent patients not receiving medical treatment. The US market for incontinence pads was estimated at $1.5 billion in 1997.

The most commonly prescribed medical treatment for OAB in western countries is the muscarinic antagonist oxybutinin. Treatment with oxybutinin is associated with a "dry mouth" side effect, which is poorly tolerated by some users. Other side effects of muscarinic antagonists include constipation, visual-accommodation abnormalities and xerothalmia (dry eyes). In Japan, propiverine is prescribed for OAB, and Flavoxate is also an important product for the treatment of urge incontinence. A second generation muscarinic M3 receptor antagonist, tolterodine, has been marketed for OAB. Tolterodine has comparable efficacy to oxybutinin but with a reduced incidence of side effects. Tolterodine is administered twice daily, where oxybutinin must be taken 2-3 times daily. Subjective efficacy rates for oxybutinin and tolterodine range from 20-50% with adverse effects (primarily dry mouth) observed in an approximately 78% and 39% of the patient population, respectively.

Estrogen and progesterone therapy has been studied for incontinence with some indication that estrogen and/or progesterone replacement therapy can partially alleviate incontinence in some women. However, there is no conclusive evidence that hormone therapy alone is sufficient to cure incontinence. Some studies have shown that hormone replacement therapy helps prevent postmenopausal recurrent urinary tract infections and improves UI. Other studies suggest alpha-adrencrgic agonists, beta-adrenergic-receptor blocking agents, cholinergic receptor-blocking compounds and cholinergic receptor-stimulating drugs may be beneficial.

Despite the availability of existing treatments, there is a major unmet and growing need for an effective and acceptable medical treatment for UI and OAB.

### Description of the Invention:

It has now been discovered that certain compounds that bind to the neurokinin 2 receptor ("NK2R") are useful for the treatment and prevention of overactive bladder ("OAB") and urinary or urethral incontinence (’’UT"). In particular, it has been discovered that the NK2R ligand (*S*)-*N*-[2-(3,4-dichlorophenyl)-4-[4-(2-oicoperhydropyrirnidin-1-yl)piperidino] butyl]-*N*-methylbenzamide is useful for the treatment and prevention of OAB and urinary or urethral incontinence.

In one aspect, the Invention provides a method comprising treating or preventing OAB or UI in a subject, particularly in a human, with an NK2R ligand. In particular the method comprises treating OAB or UI with (*S*)-*N*-[2-(3,4-dichlorophenyl)-4-[4-(2-oxoperhydropyrimidin-1-yl)piperidino]butyl]-*N*-methylbenzamide (the "Compound") and more particularly the method comprises treating with a therapeutically-effective amount of the Compound.

In a second aspect, the Invention provides the use of (*S*)-*N*-[2-(3,4-dichlorophenyl)-4-[4-(2-oxoperhydropyrimidin-1-yl)piperidino]butyl]-*N*-methylbenzamide, or a pharmaceutically-acceptable salt thereof in the preparation of a medicament for treating and/or preventing OAB or UI in mammals, and in humans in particular.

In a third aspect, the Invention provides pharmaceutically-acceptable salts of the Compound and compositions containing the Compound or pharmaceutically-acceptable salts thereof.

In another aspect, the Invention provides a method for the treatment and prevention of OAB or UI in mammals

and humans in particular comprising treating a subject in need thereof with a therapeutically-effective amount of an NK2R ligand in combination with another therapeutic agent.

In yet another aspect the Invention provides a method for the treatment and prevention of OAB or UI in mammals and humans in particular comprising treating a subject in need thereof with a therapeutically-effective amount of an NK2R ligand in combination with an estrogenic agent and/or a progestational substance, and with or without supplementation with an alpha-adrenergic agonist, beta-adrenergic receptor blocking agent, cholinergic-receptor blocking compound or a cholinergic-receptor-stimulating drug.

In a further aspect the Invention provides a pharmaceutical composition useful in the practice of the methods of the Invention comprising the Compound in accord with structural diagram I and a pharmaceutically-acceptable excipient or diluent.

In particular, it is an object of the Invention to provide a method for the treatment of OAB or UI comprising use of the Compound, (*S*)-*N*-[2-(3,4-dichlorophenyl)-4-[4-(2-oxoperhydropyrimidin-1-yl)piperidino]butyl]-*N*-methylbenzamide, having a structure in accord with structural diagram I:

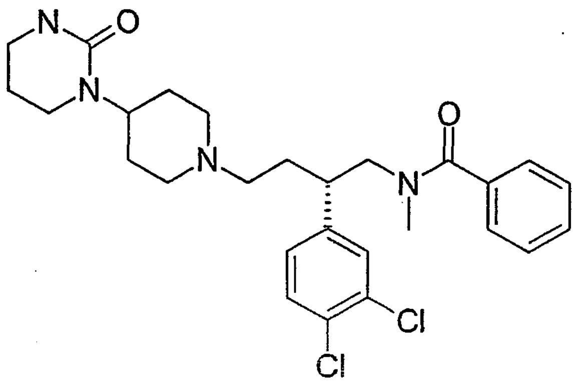

I.

It is another object of the Invention to provide a method comprising use of the Compound for the prevention of OAB or UI.

While the methods of the Invention are applicable to mammals in general they are applicable to humans in particular.

Therefore, it is an object of the Invention to provide a method for treating a human patient suffering from overactive bladder ("OAB") a term generally used, and used herein, for a syndrome that encompasses urinary urge incontinence, urgency and frequency. Treatment of urinary incontinence ("UI"), the involuntary loss of urine that results from an inability of the bladder to retain urine as a consequence of either urge (urge incontinence), or physical or mental stress (stress incontinence), is contemplated to be an object of the Invention. Treatment of OAB or UI, as contemplated herein, encompasses administration of a therapeutically-effective amount of the Compound.

Another object of the Invention is to provide the Compound in accordance with structural diagram I useful for the treatment or prevention of OAB or UI.

A further object of the Invention is to provide pharmaceutically-acceptable salts, compositions, mixtures and the like of the Compound useful for the treatment or prevention of OAB or UI.

A particular object of the invention is to provide a method of treating a human patient having OAB or UI comprising administering an effective OAB or UI treatment amount of (*S*)-*N*-[2-(3,4-dichlorophenyl)-4-[4-(2-oxoperhydropyrimidin-1-yl)piperidino]butyl]-*N*-methylbenzamide to the patient.

Another particular object of the invention is to provide a method wherein (*S*)-*N*-[2-(3,4-dichlorophenyl)-4-[4-(2-oxoperhydropyrimidin-1-yl)piperidino]butyl]-*N*-methylbenzamide is in the form of a pharmaceutically-acceptable salt thereof.

In methods of the invention phamaceutically-acceptable salts is a salt such as a chloride, sulphate, tosylate, mesylate, napsylate, besylate, phosphate, salicylate, tartrate, lactate, citrate, benzoate, succinate, acetate or a maleate.

In methods of the invention treatment is contemplated to be administered by physiologically-acceptable route, such as oral, parenteral, rectal, inhalation and insufflation.

In methods of the invention (*S*)-*N*-[2-(3,4-dichlorophenyl)-4-[4-(2-oxoperhydropyrimidin-1-yl)piperidino]butyl]-*N*-methylbenzamide is contemplated to be in a form such as a capsule, a tablet, an aqueous solution, an aqueous suspension, a non-aqueous suspension, a suppository, an aerosol or a powder.

In certain methods of the invention it is also contemplated that (*S*)-*N*-[2-(3,4-dichlorophenyl)-4-[4-(2-oxoperhydropyrimidin-1-yl)piperidino]butyl]-*N*-methylbenzamide will be administered in combination with other therapeutic agents. Such agents are contemplated to be estrogenic agents, progestational substances, alpha-adrenergic agonists, beta-adrenergic-receptor-blocking agents, cholinergic-receptor-blocking agents or cholinergic-receptor-stimulating agents. However, it will be apparent to those of skill in the art that the Compound can be co-administered with any therapeutic or prophylactic agent and/or medicament or combination thereof that is not medically-incompatible therewith.

The invention is contemplated to encompass pharmaceutical compositions comprising (S)-N-[2-(3,4-dichlorophenyl)-4-[4-(2-oxoperhydropyrimidin-1-yl)piperidino]butyl]-N-methylbenzamide together with a pharmaceutically-acceptable excipient or diluent.

The invention is also envisioned to encompass pharmaceutical compositions that include agents such as estrogenic agents, progestational substances, alpha-adrenergic agonists, beta-adrenergic-receptor-blocking agents, cholinergic-receptor-blocking agents or cholinergic-receptor-stimulating agents.

Pharmaceutical compositions contemplated to fall within the scope of the invention include those having forms such as capsules, tablets, aqueous solutions, aqueous suspensions, non-aqueous suspensions, suppositories, aerosols and powders.

In another aspect the Invention comprises pharmaceutically-advantageous salts of the Compound. Among such salts are napsylates, besylates, salicylates, tartrates, lactates, benzoates, succinates, acetates and maleates. (S)-N-[2-(3,4-dichlorophenyl)-4-[4-(2-oxoperhydropyrimidin-1-yl)piperidino]butyl]-N-methylbenzamide maleate is particularly pharmaceutically-advantageous salt.

Further aspects, objects and advantages of this Invention will become apparent to those skilled in the art upon study of the specification and the appended claims.

The Compound possesses NK2R binding properties and selectively inhibits the contraction of bladder tissues. However, it will be appreciated that when used in the treatment of OAB, UI or related disease, the Compound is contemplated to be administered as an appropriate pharmaceutical composition which comprises the Compound or a pharmaceutically-acceptable salt of the Compound, such as a chloride, sulphate, tosylaie, mesylate, napsylate, besylate, phosphate, salicylate, tartrate, lactate, citrate, benzoate, succinate, acetate, maleate, or the like, together with a pharmaceutically-acceptable diluent or carrier. Such salts are prepared by methods known to those of skill in the art. The form of a pharmaceutical composition is adapted for the particular route of administration chosen. Such forms include, for example, tablets, capsules, solutions or suspensions for oral administration; suppositories for rectal administration; sterile solutions or suspensions for administration by intravenous or intramuscular infusion or injection; aerosols or nebulizer solutions or suspensions for administration by inhalation; or powders together with pharmaceutically-acceptable solid diluents such as lactose for administration by insufflation.

For oral administration a tablet or capsule containing therapeutically-effective amount from 0.1 mg up to 250 mg (and typically 5 to 100 mg) of the Compound may conveniently be used. For administration by inhalation, the Compound will be administered to humans in a daily dose range of, for example, 5 to 100 mg, in a single dose or divided into two to four daily doses. Similarly, for intravenous or intramuscular injection or infusion a sterile solution or suspension containing up to 10% w/w (and typically 0.05 to 5% w/w) of the Compound may conveniently be used.

The dose of the Compound to be administered will necessarily be varied according to principles well known in the art, taking account of the route of administration and the severity of the condition and the size and age of the patient under treatment. General, it is contemplated that the Compound will be administered as a dose within the range of about 0.01 to about 25 mg/kg, and more particularly as a dose within the range 0.1 to 5 mg/kg. It will be understood that generally equivalent amounts of an *N*-oxide or a pharmaceutically-acceptable salt or a quaternary ammonium salt of the Compound may be used.

## Examples:

The action of the Compound as a therapeutic agent for the treatment of OAB or UI through its action to bind to NK2 receptors can be shown using suitably designed *in vitro* tests.

### *In vitro* tests:

Inhibition of NK2 Receptor-Mediated Contraction of Rabbit Pulmonary Artery:

Male New Zealand white rabbits were administered a lethal injection of Nembutal, (60 mg/kg) into a cannulated ear vein. Heparin, 0.0025 mL/kg of a 1000 U/ml solution, was injected into the ear vein prior to Nembutal in order to decrease blood coagulation. The left and right branches of the pulmonary artery were obtained from each animal. The segments, with or without endothelium, were suspended as rings in water-jacketed tissue baths maintained at 37 °C containing physiological salt solution of the following composition (mM): NaCl (119.0); KCl (4.6); CaCl2 (1.8); $MgCl_2$ (0.5); $NaH_2PO_4$ (1.0); $NaHCO_3$ (25.0); glucose (11.0); and gassed continuously with 95% $O_2$-5% $CO_2$. Initial tension

placed on each tissue was 2 gm, which was maintained throughout a 0.5 hour equilibration period. Changes in tension were measured on a Grass polygraph via Grass FT-03 force transducers.

To study NK2-mediated contraction, the vascular endothelium was removed and beta-$Ala^8$-NKA ("BANK") was used as agonist. To study NKl-mediated relaxation, the endothelium was intact and tissue contraction was induced by $3 \times 10^{-6}$ M phenylephrine, added 60 min before obtaining cumulative concentration-response effects of Ac-[$Arg^6$, $Sar^9$, $Met(O_2)^{11}$]Substance P (6-11) ("ASMSP"). Papaverine ($1 \times 10^{-3}$ M), was added at the end of the relaxation experiments to determine the maximum magnitude of relaxation (defined as 100%). The response to each concentration of ASMSP was expressed as a percentage of the total relaxation induced by papaverine. The $EC_{50}$ values for all agonists were calculated at 50% of the maximum response produced by each agonist. Apparent $K_B$ values for the compound were calculated using the standard equation: $K_B$ = [antagonist]/(dose ratio - 1), where dose ratio was equal to antilog [(-log molar $EC_{50}$ without antagonist) - (-log molar $EC_{50}$ with antagonist)]. The resulting $K_B$ values are expressed as -log molar $K_B$.

The -log molar $K_B$ values for the Compound against the NK2 receptor of rabbit pulmonary artery averaged 9.05 $\pm$ 0.08 and is similar to values obtained in human bronchus (cf. 8.62 $\pm$ 0.14). In addition, the Compound caused minor antagonism of relaxation of the rabbit pulmonary artery induced by ASMSP. However, the -log molar $K_B$ value for the antagonist against the NK1 receptor-mediated response is 1,096-fold smaller than against the NK2 receptor-mediated response in this tissue. These results demonstrate that the Compound is a highly potent and selective antagonist of the tachykinin NK2 receptor.

Neurokinin Receptor Binding Assay:

The ability of a substance to antagonize neurokinin A ("NKA") binding at the NK2 receptor may be demonstrated with an assay using the human NK2 receptor expressed in Mouse Erythroleukemia ("MEL") cells. MEL cell membranes ("MELM") bear high-affinity and selective NK2 receptors which bind NKA and the inhibition of the binding ofNKA by antagonists may be demonstrated as described in U.S. Patent 5,567,700, Test A, the disclosure of which is incorporated herein in its entirety.

MELM were prepared from MEL cells containing high-affinity NK2 receptors according to a published protocol (D. Aharony, *et al., Mol. Pharmacol.* 45:9-19, 1994) with the following modifications: (a) iodoacetamide (1 mM) was included in the homogenization buffer; (b) homogenization was as published but only one time for a shorter period of 10 seconds and at speed setting 10; and (c) the equilibration step with KCI/EDTA was not performed.

In a typical preparation, binding of $^3$H-NKA (2.5 nM) to MELM was highly specific (96 + 1%) and linearly dependent on the protein concentration, with significant binding detected as low as 26 $\mu$g protein/mL. Equilibrium-competition experiments demonstrated binding to high-affinity, high-density receptors with $K_D$ = 4.2 $\pm$ 0.35 nM, $B_{max}$ = 6257 $\pm$ 257 fmol/mg protein.

The radio ligand 3H-neurokinin A ("$^3$H-NKA") as [4,5-$^3$H-$Leu^9$]-NKA (typical specific activity, 117 Ci/mmol) was obtained by custom synthesis from Cambridge Research Biochemicals and is >95% pure. Repeated HPLC analysis demonstrated that the ligand was stable when stored in silanized vials with 0.2% mercaptoethanol, under argon. No degradation or metabolism of the ligand was apparent in the receptor-binding assay.

The assay was carried out using an incubation buffer consisting of 50 mM Tris HCl (pH 7.4), 5 mM $Mg^{++}$, 100 $\mu$M thiorphan, 1 nM $^3$H-NKA, 0.02% (w:v) BSA, 30 mM $K^+$, and 300 $\mu$M dithiothreitol; and the concentration of membrane protein was held at approximately 0.05-0.025 mg per tube. Nonspecific binding was routinely defined with 1 $\mu$M NKA. Each tube received the following: 150 $\mu$L incubation buffer, 20 $\mu$L $^3$H-NKA, 20 $\mu$L Compound, NKA or buffer as appropriate, and 125 $\mu$L membrane suspension. The reaction was initiated by the addition of the membranes. The tubes are incubated for 60 min at 25 °C in a shaking water bath and the reaction terminated by washing the tubes with 10 mL of ice-cold 50 mM Tris HCl using a Brandel cell harvesting system to collect the membranes. Membranes were collected using Whatman GF/B filters presoaked at least 4 hours at room temperature in 0.01% (w:v) polyethylenimine. The filters were placed in scintillation vials and read in a Beckman LS 6000LL Scintillation Counter. The binding constant, Ki, was calculated by standard methods and was typically the mean of several such determinations.

The specificity of inhibition by the Compound was shown by inhibiting the binding of tritiated NKA at the NK2 receptor compared to inhibition of binding of a tritiated derivative of SP in a tissue preparation selective for NK1 receptors. The Ki, defined as the concentration of Compound at which 50% inhibition of binding of NKA was achieved, was $1.25 \times 10^{-9}$M. 50% inhibition of binding of SP was achieved at a Compound concentration of $1.87 \times 10^{-7}$M.

***In vivo* tests:**

It will be further appreciated by those skilled in the art that the efficacy of the Invention can be demonstrated by standard assays *in vivo.* What follows describes certain such standard tests.

*In Vivo* Pulmonary Pharmacology of The Compound Against Dyspnea:

Dyspnea (labored abdominal breathing) was induced in conscious guinea pigs by aerosol administration of BANK, as previously described (Kusner, et al. *Euro. J. Pharmacol.* 210:299-306,1992). All animals were pretreated with atropine, 10 mg/kg ip 45 minutes; indomethacin, 10 mg/kg ip 30 minutes; propranolol, 5 mg/kg ip 30 minutes; and thiorphan, 1 mg/ml aerosol for 5 minutes duration, starting 15 minutes prior to challenge in order to block muscarinic receptors, cyclooxygenase, beta-adrenergic receptors, and neutral endopeptidase (EC 3.4.24.11), respectively. Animals were pretreated with Compound or vehicle at appropriate times before challenge with aerosolized BANK for 780 seconds. BANK was administered to six animals simultaneously by being aerosolized with a DeVilbiss ultrasonic nebulizer into an air stream flowing into a Plexiglas chamber at a rate of 2 liter/min. The time required for dyspnea to occur was recorded for each animal. Animals that did not undergo dyspnea during the 780 second exposure were deemed to have been fully protected from the effects of the agonist by the test compound. Vehicle pretreated control animals were included in each experiment. Results are reported in % protection values where:

$$\%\ \text{protection} = \frac{(\text{drug time - mean control time}) \times 100}{(\text{maximal aerosol time - mean control time})}$$

The Compound administered intravenously 30 min prior to challenge caused dose-dependent inhibition with an $ED_{50}$ of 0.13 μmol/kg (0.07 mg/kg). Maximum efficacy of 95% was obtained with a 0.3 μmol/kg challenge. Likewise, upon oral administration 120 min before challenge, the Compound caused dose-dependent inhibition with an $ED_{50}$ of 0.75 μmol/kg (0.39 mg/kg). Maximum efficacy of 100% was obtained with an oral dose of 3.0 μmol/kg of the Compound. The ratio of oral to intravenous $ED_{50}$ values was 5.8. Duration of action studies performed using a 2 mg/kg dose of the compound demonstrated a protection half-life against BANK-induced dyspnea of 146 min.

*In Vivo* Pulmonary Mechanics in Anesthetized Guinea Pigs:

Guinea pigs were anesthetized with urethane administered by intraperitoneal injection (1.5 g/kg) and surgically-prepared for the recording of pulmonary mechanics as described previously (C.K. Buckner, et al. *J. Pharmacol. Exp. Ther.* 267:1168-1175, 1993). Flow was obtained via a Fleisch (no 0000) pneumo-tachograph attached between the end of a tracheal cannula and a Validyne differential pressure transducer. Flow was integrated to provide tidal volume. Transpulmonary pressure was obtained by measuring the pressure difference between an intrapleural cannula placed in the 5th or 6th intercostal space and a sidearm adapter of the tracheal cannula with a Validyne differential pressure transducer. The Buxco Pulmonary Mechanics computer (model 6) calculated pulmonary resistance ($R_P$) and dynamic lung compliance ($C_{dyn}$) on a breath-by-breath basis. Analog computer outputs were digitized and printed on a Texas Instruments printer.

All drugs were administered intravenously and dose-response effects of agonists were obtained by measuring the peak response after each bolus injection. Maximum response to an agonist was defined as zero conductance ($G_L$, the reciprocal of $R_P$). The peak value of $G_L$ as expressed as a percentage of the baseline value before agonist was added and the $ED_{50}$ value was calculated as the dose resulting in a reduction of $G_L$ to 50% of baseline. All $ED_{50}$ values were converted to the negative logarithm and expressed as -log $ED_{50}$. Only one agonist dose-response curve was obtained in each animal. Pretreatment time with the Compound before agonist administration was 30 min. The selective NK1 receptor antagonist SR 140333, (3 μmol/kg) was intravenously administered 25 min before BANK was used to cause bronchoconstriction.

The Compound was administered intravenously at 10 mmol/kg 30 min before constructing a dose-response relationship with intravenously administered BANK. Under these experimental conditions, the Compound caused an approximate 196-fold rightward shift of the response curves for BANK. Oral administration of the Compound (10 mmol/kg) 120 min before BANK, caused an approximate 481-fold rightward shift in the BANK-induced dose-response curve. The magnitude of shift was similar for all responses measured.

An *in vivo* test which is complementary to the above described tests can be used to ascertain if a test compound is active when dosed intravenously or orally, and whether a test compound exhibits inhibitory effects on bladder contractile responses.

*In vivo* Bladder Contraction Inhibition in Rats:

Female Wistar rats weighing 200-300 grams were anesthetized by intramuscular administration of ketamine/ xylazine mixture (3/10 mg/kg, respectively). For each rat, the abdominal region and the front of the neck was shaved. For jugular catheterization, the right jugular vein was exposed via a small ventral cervical incision. The catheter, filled with 0.9% saline, was introduced approximately 2.0 cm into the vein. The distal end of the catheter was connected to a syringe for administration of test compound where appropriate. For bladder catheterization, the bladder was exposed through a midline abdominal incision. The ureters were tied with 4-0 silk suture approximately 2 cm above the bladder. Small incisions were made in each ureter above the ligature to allow drainage from the kidneys. A catheter was passed through the proximal urethra and bladder sphincter into the bladder lumen. The catheter was flushed with saline and patency was noted. The bladder was manually emptied and inflated with 0.3 mL saline. The cannula was attached to a Gould p23 ID pressure transducer for recording changes in bladder pressure.

An equilibration period of approximately 15 min was allowed for stabilization of the animals. Each rat was pretreated with intravenous administration of 10 mg/kg thiorphan to inhibit neutral endopeptidase 3.4.24.11. Thiorphan was administered 15 min before exposure to the agonist, BANK.

For intravenous studies, animals were administered 1-3 nmol/kg BANK intravenously and bladder contraction were recorded as an increase in intravesical bladder pressure on a Grass 7D Polygraph. The response was allowed to decay during a 15 min equilibration period before intravenous administration of the compound (0.2-5 μmol/kg, 5% PEG 400-saline vehicle). Subsequently, an additional 15 min equilibration period elapsed before administration of an equivalent dose of BANK was repeated. Preliminary studies were performed to establish equivalence of bladder contractile responses to multiple administrations of BANK. Inhibitory effects of the Compound were calculated as the percentage difference between the response to BANK in the presence and_absence of the Compound.

To establish the dose-response effects of oral compound, rats were administered the antagonist (0.2-5 μmol/kg, 5% PEG 400-saline vehicle) by gavage 1 hour before administration of BANK. Duration of action studies were performed following oral administration of the Compound (1.2 μmol/kg, 5% PEG 400-saline vehicle) at the times noted prior to administration of BANK. Responses were calculated as the percentage difference between the response to BANK in the presence of Compound compared with sham-treated controls.

For all studies, each animal was administered a single dose of Compound. Experimental results were expressed as the mean plus or minus the Standard Error of the Mean ("± S.E.M") % change from basal level.

The Compound is active and selective in this test when dosed intravenously and orally within the range of 0.2 and 5 μmol/kg body weight. When administered intravenously, an inhibitory dose of the Compound that caused 50% attenuation of the BANK-induced contraction was 0.13 μmol/kg or approximately 0.07 mg/kg. The maximum efficacy of the Compound at 3.5 μmol/kg (1.8 mg/kg) against intravenous BANK (3 nmol/kg) was approximately 100%.

When administered orally, an inhibitory dose of the Compound that caused 50% attenuation of the BANK-induced contraction was 1.0 μmol/kg or approximately 0.5 mg/kg. The maximum efficacy achieved with the Compound at 5 μmol/kg (2.5 mg/kg) was about 80%. Duration of action of the Compound was demonstrated to be about 6 hr with an approximate half-life of 3.5 hr.

The Compound has not been found to show any indication of any untoward side-effects in laboratory test animals at several multiples of the minimum effective dose.

As used herein, unless stated otherwise:

(i) temperatures are given in degrees Celsius ("°C"); operations were carried out at room or ambient temperature, that is, at a temperature in the range of 18-25 °C;

(ii) organic solutions were dried over anhydrous $MgSO_4$; evaporation of solvent was carried out using a rotary evaporator under reduced pressure (600-4000 pascals; 4.5-30 mm Hg) with a bath temperature of up to 60 °C;

(iii) chromatography, means flash chromatography; reversed phase chromatography, means flash chromatography over octadecylsilane ("ODS") coated support having a particle diameter of 32-74 μ, known as "PREP-40-ODS" (Art 731740-100 from Bodman Chemicals, Aston, PA, USA); Thin layer chromatography ("TLC") was carried out on silica gel plates;

(iv) in general, the course of reactions was followed by TLC and reaction times are given for illustration only;

(v) melting points are uncorrected and "dec" indicates decomposition; the melting points given are those obtained for the materials prepared as described; polymorphism may result in isolation of materials with different melting points in some preparations;

(vi) final products had satisfactory proton nuclear magnetic resonance ("NMR") spectra;

(vii) yields are given for illustration only and are not necessarily those which may be obtained by diligent process development; preparations were repeated if more material was required;

(viii) when given, NMR data is in the form of delta values for major diagnostic protons, given in parts per million ("ppm") relative to tetramethylsilane ("TMS") as an internal standard, determined at 300 MHz using perdeuterio

dimethyl sulfoxide ("DMSO-d6") as solvent; conventional abbreviations for signal shape are used; coupling constants (J) are given in Hz; Ar designates an aromatic proton when such an assignment is made;

(ix) chemical symbols have their usual meanings; SI units and symbols are used;

(x) reduced pressures are given as absolute pressures in pascals ("Pa"); elevated pressures are given as gauge pressures in bars;

(xi) solvent ratios are given in volume:volume ("v/v") terms; and

(xii) mass spectra ("MS") were run with an electron energy of 70 electron volts in the electron impact ("EI") mode using a direct exposure probe; where indicated ionization was effected by chemical ionization ("CI") or fast atom bombardment ("FAB"); values for m/z are given; generally, only ions which indicate the parent mass are reported.

**Chemical Examples:**

Example 1:

(*S*)-*N*-[2-(3,4-Dichlorophenyl)-4-[4-(2-oxoperhydro-pyrimidin-1-yl)piperidino]butyl]-*N*-methylbenzamide dihydrochloride.

A stirred solution of (*S*)-*N*-[-4-[4-(3-aminopropylamino)-piperidino]-2-(3,4-dichlorophenyl)butyl]-*N*-methylbenzamide (0.356 g) and 1,1'-carbonyldiimidazole (0.157 g) in chloroform (6 mL) was heated at reflux for 2 hours. The reaction mixture was diluted with dichloromethane, washed (aqueous sodium bicarbonate), dried, evaporated, and purified by chromatography, with dichloromethane:methanol (gradient 98:2, 90:10) as eluent. The resulting material was dissolved in dichloromethane, precipitated as the hydrochloride salt with ethereal hydrogen chloride, evaporated, and placed under high vacuum overnight to give the title compound as a white solid. MS: m/z=517(M+1); Analysis for $C_{27}H_{34}Cl_2N_4O_2 \cdot 2.60\ HCl \cdot 0.13\ (C_2H_5)_2O$: Calculated: C, 53.14; H, 6.14; N, 9.00; Found: C, 53.14; H, 6.31; N, 9.16. The intermediate, (*S*)-*N*-[4-[4-(3-aminopropylamino)piperidino]-2-(3,4-dichlorophenyl)butyl]-N-methylbenzamide was prepared as follows:

1a. 1-Benzyloxycarbonyl-4-(3-aminopropylamino)piperidine.

1-Benzyloxycarbonyl-4-oxo-piperidine (12.0 g) in methanol (72 mL) was added to a stirred solution of 1,3-diaminopropane (5.2 mL) and acetic acid (8.8 mL) in methanol (72 mL). After 15 minutes, sodium cyanoborohydride (9.7 g) in methanol (72 mL) was added in a single portion. After being stirred overnight, the reaction mixture was evaporated; and the residue was dissolved in 1 N hydrochloric acid (100 mL). Concentrated hydrochloric acid was added dropwise and stirring was continued until the evolution of gas ceased. The acidic aqueous mixture was washed with dichloromethane, basified to pH 10 with 10 N sodium hydroxide, and extracted with dichloromethane. The dichloromethane extracts were dried and evaporated to give the title compound as a viscous oil. MS: m/z=292(M+1); NMR ($CD_3OD$): 7.34 (m,5), 5.10 (s,2), 4.13 (m,2), 2.86 (m,2), 2.65 (m,5), 1.90 (m,2), 1.65 (m,2), 1.23 (m,2).

1b. 1-Benzyloxycarbonyl-4-[2,2,2-trifluoroacetyl)-[3-(2,2,2-trifluoroacetylamino) propyl]amino]piperidine.

Trifluoroacetic anhydride (10.5 mL) was added to a solution 1-benzyloxy-carbonyl-4-(3-aminopropylamino) piperidine(7.5 g) and triethylamine (8.3 mL) in chloroform (90 mL) at 0°C. After being stirred overnight, the reaction mixture was diluted with dichloromethane, washed (1 N hydrochloric acid, aqueous sodium bicarbonate), dried, evaporated, and purified by chromatography, with dichloromethane:methanol (98:2) as eluent, to give the trifluoroacetylated piperidine as a viscous oil. NMR: 7.36 (m,5), 5.14 (s,2), 4.35 (m,2), 3.93 (m,1), 3.35 (m,4), 2.83 (m,2), 1.87-1.74 (m,6); MS: m/z=484(M+1).

1c. 4-[(2,2,2-Trifluoroacetyl)[3-(2,2,2-trifluoroacetylamino)propyl]amino]piperidine.

A solution of 1-benzyloxycarbonyl-4-[(2,2,2-trifluoroacetyl)-[3-(2,2,2-trifluoroacetyl-amino)propyl]amino]piperidine(1.85 g) and 20% palladium hydroxide on carbon (0.340 g) in ethanol (30 mL) was stirred overnight under 1 bar of hydrogen. The reaction mixture was filtered through diatomaceous earth and the filtrate was evaporated to give the title compound (0.950 g) as a viscous oil. NMR ($CD_3OD$): 4.39 (m,1), 3.98 (m,1), 3.30 (m,3), 2.95 (m,1), 2.82 (m,1), 2.65 (m,2), 2.01 (m,2), 1.75 (m,2), 1.32 (m,2); MS: m/z=350(M+1).

1d. (S)-*N*-[2-(3,4-Dichlorophenyl)-4-[4-[(2,2,2-trifluoroacetyl)-[2-(2,2,2-trifluoroacetyl-amino)ethyl]amino]piperidino] butyl]-*N*-methylbenzamide.

(S)-*N*-[2-(3,4-Dichlorophenyl)-4-oxobutyl]-*N*-methylbenzamide (0.823 g) in methanol (4 mL) was added to a solution of 4-[(2,2,2-trifluoroacetyl) -[3-(2,2,2-trifluoroacetyl-amino)propyl]amino] piperidine (0.600 g) and acetic acid (0.20 mL) in methanol (8 mL). After 5 minutes, sodium cyanoborohydride (0.220 g) in methanol (4 mL) was added in a single portion. After being stirred for 3 hours, the reaction mixture was diluted with aqueous sodium bicarbonate, stirred for 30 minutes, and extracted with dichloromethane. The organic extracts were dried, evaporated, and purified by chromatography, with dichloromethane:methanol (gradient 98:2, 90:10) as eluent. The resulting material was dissolved in dichloromethane, precipitated as the hydrochloride salt with ethereal hydrogen chloride, evaporated, and placed under high vacuum overnight to give the title compound as a white solid. MS: m/z=683(M+1).

1e. (*S*)-*N*-[4-[4-(3-Aminopropylamino)piperidino]-2-(3,4-dichlorophenyl)butyl]-*N*-methylbenzamide.

A solution of the crude (S)-*N*-[2-(3,4-dichlorophenyl)-4-[4-[(2,2,2-trifluoroacetyl)[3-(2,2,2-trifluoroacetylamino) propyl]amino]-piperidino]butyl]-N-methylbenzamide (2.5 g) in 20% aqueous potassium hydroxide (8.5 mL) and methanol (11 mL) was stirred for 1 hour. The reaction mixture was acidified to pH 2 with 1 N hydrochloric acid and washed 3 times with dichloromethane. The aqueous phase was then basified to pH 10 with 10 N sodium hydroxide and extracted with dichloromethane. The extracts were dried and evaporated to give the title compound as a viscous oil. MS: m/z=491(M+1).

<u>Example 2:</u>

(*S*)-*N*-[2-(3,4-dichlorophenyl)-4-[4-(2-oxoperhydropyrimidin-1-yl)piperidino]butyl]-*N*-methylbenzamide dihydrochloride:

A stirred solution of (*S*)-*N*-[-4-[4-(3-aminopropylamino)piperidino]-2-(3,4-dichlorophenyl)butyl]-*N*-methylbenzamide, (0.356 g) and 1,1'-carbonyldiimidazole (0.157 g) in chloroform (6 mL) was heated at reflux for 2 hours. The reaction mixture was diluted with dichloromethane, washed (aqueous sodium bicarbonate), dried, evaporated, and chromatographed, with dichloromethane:methanol (gradient 98:2, 90:10) as eluent. The resulting material was dissolved in dichloromethane, precipitated as the hydrochloride salt with ethereal hydrogen chloride, evaporated, and placed under high vacuum overnight to give the title compound as a white solid; MS: m/z = 517(M+1); Analysis for $C_{27}H_{34}Cl_2N_4O_2 \cdot 2.60$ $HCl \cdot 0.13$ $(C_2H_5)_2O$: Calculated: C, 53.14; H, 6.14; N, 9.00; Found: C, 53.14; H, 6.31; N, 9.16.

<u>Example 3:</u>

(*S*)-*N*-[2-(3,4-dichlorophenyl)-4-[4-(2-oxoperhydropyrimidin-1-yl)piperidino]butyl]-*N*-methylbenzamide maleate salt:

A stirred, 50 °C solution of (*S*)-*N*-[2-(3,4-dichlorophenyl)-4-[4-(2-oxoperhydropyrimidin-1-yl)piperidino] butyl]-*N*-methylbenzamide (22.29 g) in ethyl acetate (446 mL) was treated dropwise with a solution of maleic acid (5 g) in absolute ethanol (44.6 mL). Following completion of maleic acid addition, the resulting slurry was stirred for 30 min at 50 °C, slowly cooled to room temperature over 4 h, then stirred at room temperature overnight. The suspension was filtered, washed with ethyl acetate and dried *in vacuo* at 40 °C to afford the title compound as a white crystalline powder.

**Claims**

**1.** The use of (*S*)-*N*-[2-(3,4-dichlorophenyl)-4-[4-(2-oxoperhydropyrimidin-1-yl)piperidino]butyl]-*N*-methylbenzamide or a pharmaceutically-acceptable salt thereof in the preparation of a medicament for treating or preventing overactive bladder or urinary incontinence.

**2.** The use according to Claim 1, of a pharmaceutically-acceptable salt selected from a chloride, a sulphate, a tosylate, a mesylate, a napsylate, a besylate, a phosphate, a salicylate, a tartrate, a lactate, a citrate, a benzoate, a succinate, and acetate or a maleate.

**3.** The use according to Claim 1, in the preparation of a medicament suitable for co-administration with one or more other medically-compatible therapeutic agents.

**4.** The use according to Claim 3, wherein said other medically-compatible therapeutic agents are selected from, an estrogenic agent, a progestational substance, an alpha-adrenergic agonist, a beta-adrenergic receptor blocking

agent, a cholinergic-receptor blocking compound or a cholinergic-receptor-stimulating drug.

5. The use according to Claim 1, in the preparation of a medicament suitable for administration orally, parenterally, rectally, by inhalation or by insufflation.

6. The use according to Claim 1, in the preparation of a medicament suitable for oral administration.

7. The use according to Claim 1, in the preparation of a medicament comprising a tablet or capsule containing about 0.1 mg up to about 250 mg of said compound or pharmacsutically-acceptable salt thereof.

8. The use according to Claim 7, comprising a tablet or capsule containing about 5 mg to about 100 mg of said compound or pharmaceutically-acceptable salt thereof.

9. The use according to Claim 1, in the preparation of a medicament comprising about 0.1 mg to about 250 mg of said compound suitable for administration by inhalation as a single dose or divided into two, three or four daily doses.

10. The use according to Claim 9, in the preparation of a medicament comprising about 5 mg to about 100 mg of said compound suitable for administration by inhalation as a single dose or divided into two, three or four daily doses.

11. The use according to Claim 1, in the preparation of a medicament suitable for administration as a capsule, a tablet, an aqueous solution, an aqueous suspension, a non-aqueous suspension, a suppository, an aerosol or a powder.

**Patentansprüche**

1. Verwendung (*S*)-*N*-[2-(3,4-Dichlorphenyl)-4-[4-2-oxoperhydropyrimidin-1-yl)piperidino]butyl]-*N*-methylbenzamid oder eines pharmazeutisch annehmbaren Salzes davon bei der Herstellung eines Medikaments zur Behandlung oder Prävention von hyperaktiver Blase oder Harninkontinenz.

2. Verwendung nach Anspruch 1 eines pharmazeutisch annehmbaren Salzes ausgewählt aus einem Chlorid, einem Sulfat, einem Tosylat, einem Mesylat, einem Napsylat, einem Besylat, einem Phosphat, einem Salicylat, einem Tartrat, einem Lactat, einem Citrat, einem Benzoat, einem Succinat, einem Acetat oder einem Maleat.

3. Verwendung nach Anspruch 1 bei der Herstellung eines Medikaments, das für die gemeinsame Verabreichung mit einem oder mehreren anderen, medizinisch kompatiblen therapeutischen Mitteln geeignet ist.

4. Verwendung nach Anspruch 3, wobei die anderen, medizinisch kompatiblen therapeutischen Mittel ausgewählt sind aus einem östrogenen Mittel, einer progestationalen Substanz, einem alpha-adrenergen Agonisten, einem beta-adrenergerezeptorenblockierenden Mittel, einer cholinergerezeptorenblockierenden Verbindung oder einem cholinergerezeptorenstimulierenden Arzneimittel.

5. Verwendung nach Anspruch 1 bei der Herstellung eines Medikaments, das sich für die orale parenterale oder rektale Verabreichung oder die Verabreichung durch Inhalation oder durch Insufflation eignet.

6. Verwendung nach Anspruch 1 bei der Herstellung eines Medikaments, das sich für die orale Verabreichung eignet.

7. Verwendung nach Anspruch 1 bei der Herstellung eines Medikaments, das eine Tablette oder Kapsel umfaßt, die etwa 0,1 mg bis etwa 250 mg der Verbindung oder eines pharmazeutisch annehmbaren Salzes davon enthält.

8. Verwendung nach Anspruch 7, umfassend eine Tablette oder Kapsel, die etwa 5 mg bis etwa 100 mg der Verbindung oder eines pharmazeutisch annehmbaren Salzes davon enthält.

9. Verwendung nach Anspruch 1 bei der Herstellung eines Medikaments, umfassend etwa 0,1 mg bis etwa 250 mg der für die Verabreichung durch Inhalation geeigneten Verbindung als Einzeldosis oder aufgeteilt in zwei, drei oder vier Tagesdosen.

10. Verwendung nach Anspruch 9 bei der Herstellung eines Medikaments, umfassend etwa 5 mg bis etwa 100 mg der für die Verabreichung durch Inhalation geeigneten Verbindung als Einzeldosis oder aufgeteilt in zwei, drei oder vier

Tagesdosen.

**11.** Verwendung nach Anspruch 1 bei der Herstellung eines Medikaments, das sich für die Verabreichung als eine Kapsel, eine Tablette, eine wäßrige Lösung, eine wäßrige Suspension, eine nichtwäßrige Suspension, ein Zäpfchen, ein Aerosol oder ein Pulver eignet.

**Revendications**

**1.** Utilisation du (*S*)-*N*-(2-(3,4-dichlorophényl)-4-[4-(2-oxoperhydropyrimidin-1-yl)pipéridino]butyl]-*N*-méthylbenzamide ou d'un sel pharmaceutiquement acceptable de celui-ci pour la préparation d'un médicament destiné au traitement ou à la prévention d'une suractivité de la vessie ou de l'incontinence urinaire.

**2.** Utilisation selon la revendication 1, d'un sel pharmaceutiquement acceptable choisi parmi un chlorure, un sulfate, un tosylate, un mésylate, un napsylate, un bésylate, un phosphate, un salicylate, un tartrate, un lactate, un citrate, un benzoate, un succinate, et un acétate ou un maléate.

**3.** Utilisation selon la revendication 1, pour la préparation d'un médicament approprié pour une co-administration avec un ou plusieurs autres agents thérapeutiques compatibles du point de vue médical.

**4.** Utilisation selon la revendication 3, dans laquelle lesdits autres agents thérapeutiques compatibles du point de vue médical sont choisis parmi un agent oestrogénique, une substance progestative, un agoniste alpha-adrénergique, un agent bloqueur de récepteur bêta-adrénergique, un composé bloqueur de récepteur cholinergique ou un médicament stimulant un récepteur cholinergique.

**5.** Utilisation selon la revendication 1, pour la préparation d'un médicament approprié pour une administration par voie orale, parentérale, rectale, par inhalation ou par insufflation.

**6.** Utilisation selon la revendication 1, pour la préparation d'un médicament approprié pour une administration par voie orale.

**7.** Utilisation selon la revendication 1, pour la préparation d'un médicament comprenant un comprimé ou une capsule contenant d'environ 0,1 mg à environ 250 mg dudit composé ou sel pharmaceutiquement acceptable de celui-ci.

**8.** Utilisation selon la revendication 7, comprenant un comprimé ou une capsule contenant d'environ 5 mg à environ 100 mg dudit composé ou sel pharmaceutiquement acceptable de celui-ci.

**9.** Utilisation selon la revendication 1, pour la préparation d'un médicament comprenant d'environ 0,1 mg à environ 250 mg dudit composé approprié pour une administration par inhalation en tant que dose unique ou divisé en deux, trois ou quatre doses journalières.

**10.** Utilisation selon la revendication 9, pour la préparation d'un médicament comprenant d'environ 5 mg à environ 100 mg dudit composé approprié pour une administration par inhalation en tant que dose unique ou divisé en deux, trois ou quatre doses journalières.

**11.** Utilisation selon la revendication 1, pour la préparation d'un médicament approprié pour une administration sous forme d'une capsule, d'un comprimé, d'une solution aqueuse, d'une suspension aqueuse, d'une suspension non aqueuse, d'un suppositoire, d'un aérosol ou d'une poudre.